# EUROPEAN PATENT SPECIFICATION

(11) **EP 1 533 369 B1**
(45) Date of publication and mention of the grant of the patent: **23.03.2011**
(21) Application number: 03026432.9
(22) Date of filing: 19.11.2003
(51) Int. Cl.: C12N 7/00, C12N 7/02, A61K 35/76

(54) **Isolated phages and their use as disinfectant in food or for sanitation of factory environment**
Isolierte Bakteriophage und ihre Verwendung in Lebensmitteln oder zur Sanierung der Fabrik-Umgebung
Bacteriophages isolées et leur utilisation dans la nouriture ou pour l'assainissement en millieu industrielle

(43) Date of publication of application: 25.05.2005
(73) Proprietor: Nestec S.A., 1800 Vevey (CH)
(72) Inventor: Breeuwer, Pieter, 1023 Crissier (CH); Boissin-Delaporte, Catherine, 01170 Echevenex (FR); Joosten, Henricus, 1074 Mollie-Margot (CH); Lardeau, Annick, 1010 Lausanne (CH)
(74) Representative: Chautard, Cécile

(56) References cited:
- WO-A-03/054173
- US-B1- 6 322 783
- GRIMONT F ET AL: "DNA relatedness among bacteriophages of the morphological group C3" CURRENT MICROBIOLOGY 1981 UNITED STATES, vol. 6, no. 2, 1981, pages 65-69, XP009027844
- GRIMONT F ET AL: "Characteristics of five bacteriophages of yellow-pigmented enterobacteria" CURRENT MICROBIOLOGY 1981 UNITED STATES, vol. 5, no. 1, 1981, pages 61-66, XP009027826
- BREEUWER P ET AL: "Desiccation and heat tolerance of Enterobacter sakazakii." JOURNAL OF APPLIED MICROBIOLOGY, vol. 95, no. 5, 2003, pages 967-973, XP002273937 ISSN: 1364-5072

## Description

### Field of the invention

The present invention relates to phage isolates having a strong lytic activity against *Enterobacter sakazakii* strains and their use as anti-microbial agent in food products, in particular infant formula, and for sanitation of factory environments.

### Background of the invention

Enterobacteria represent a dominant portion of the "recontamination flora" of dairy products made from pasteurised milk. Their presence often indicates inadequate sanitization and/or faulty construction of the processing equipment. Members of the genus *Enterobacter* are frequently found in dried products such as milk powder.

Among the *Enterobacteria, E. sakazakii* can cause serious infections especially among very young. It has been implicated in a rare but severe form of neonatal meningitis among neonates and infants, with dried-infant formula being implicated as the mode of transmission.

Aseptic manufacturing of dried infant formula is not feasible because the costs for the production will be too elevated. Therefore, the manufacturers rely on the use of disinfectants and specific hygienic work zones during processing to provide safe products. Nevertheless, low numbers of bacteria can grow to considerable counts when a reconstituted infant formula is not consumed rapidly and kept at ambient temperature. This may render the product potentially dangerous for the infant consuming it.

In view of the deficiencies in the prior art, the problem of the present invention resides in providing a food, in particular milk based products such as infant formulas, which may be safe even after storage in conditions favouring the growth of *E*. *sakazakii.* Also, the invention aims to develop a safe agent that can be used for decontamination of *E. sakazakii* in any food product and for sanitation of factory environments.

This problem has been solved by providing bacteriophage preparations which, different to bacteriophages known in the prior art (Grimon F. et al., Current Microbiology, vol. 6, no. 2, 1981, pages 65-69 and Current Microbiology, vol. 5, no. 2, 1981 pages 61-66), have substantial lytic potential towards *E*. *sakazakii,* and are safe and non-toxic.

### Summary of the invention

It is an object of the present invention to develop new and useful phage isolates with substantial lytic potential towards *E. sakazakii* and which can be used alone or in cocktail to prevent or to treat food products and factory environments from contaminations caused by *E*. *sakazakii,* in particular infant formulas.

It is thus another object of the present invention to provide a food product that contains phage isolates selected to lyse E.sakazakii., in particular a milk based formula, a nutritional composition, a pet food product, a dietary supplement or any food at risk of contamination by *E. sakazakii.*

It is a further object of the present invention to provide an agent for decontamination of food product and sanitation of factory environment, which comprises an effective amount of at least one phage isolate or cocktail as described above.

In another embodiment the present invention relates to the use of at least one phage isolate or cocktail as described above, for decontamination of food products and sanitation of factory environment or in the preparation of a composition intended to prevent or treat infections caused by *E. sakazakii* in humans or animals.

Further described is a method of decontamination of food products or sanitation of factory environment, which comprises using at least one bacteriophage preparation with substantial lytic potential towards *E. sakazaki* as described above.

A method for selecting phage isolate with substantial lytic potential towards *E*. *sakazakii* as described above, is also described

A major advantage of the present invention is that it provides for a decontaminant having a high specificity for *E*. *sakazakii.* It may prevent contamination and outgrowth of *E. sakazakii* in any food product, being particularly useful for milk based products such as infant formulas. It can also be efficient for the treatment or prevention of infections caused by *E. sakazakii* in humans or animals.

Another advantage of the present invention is that it provides an agent which helps to disinfect, clean and decontaminate working surfaces or processing equipment in factories, as well as floors; walls and the like.

Another advantage of the present invention is that it provides an agent that is environmentally safe and non-toxic for humans.

### Detailed Description of the Invention

Within the following description, the term "FSM-phage" designates the Food Safety Microbiology bacteriophage collection (Nestlé Research Centre, Vers-chez-les-Blanc, Lausanne, Switzerland).

An agent for decontamination of food and sanitation of factory environment, comprises at least one phage isolate with substantial lytic potential towards *E. sakazakii,* is concerned.

The phages according to the present invention may be obtained by screening candidate bacteriophages for those which infect *E. sakazakii* strains using the spot assay test (Pelczar, M.J., E.C.S. Chan, and N.R. Krieg. Microbiology concepts and applications, Chapter 16 Viruses: cultivation methods, pathogencity p.436-452, McGraw-Hill, Inc. New York 1993), or in liquid culture (H.W. Ackermann and M.S. DuBow. Viruses of Prokaryotes volume 1. Chapter 6. Description and identification of new phages p. 103-142. CRC Press Inc, Boca Raton, Florida, USA.). The method for selecting the Bacteriophages is detailled in example 1. Preferably, the phage isolate having the ability to infect:
i) at least 9 bacterial strains of *E.sakazakii* from the group consisting of FSM-16; FSM-33; FSM-261; FSM-265; FSM-266, FSM-269; FSM-270; FSM-271; FSM-272; FSM-273; FSM-274; FSM-280; FSM-281; FSM-284; FSM-286; FSM-288; FSM-290; FSM-292; FSM-297; FSM-298; FSM-300; FSM-303; FSM-305; FSM-308; FSM-309; FSM-311; FSM-313; FSM-314; FSM-316; FSM-318; FSM-322; FSM-323; FSM-1360; FSM-1387-2NL; FSM-MC7; FSM-MC8; FSM-MC9; FSM-MM10 and FSM-MM11, on solid medium and,
ii) at least 9 bacterial strains of *E.sakazakii* from the group consisting of FSM-16; FSM-33; FSM-261; FSM-265; FSM-266, FSM-269; FSM-270; FSM-271; FSM-272; FSM-273; FSM-274; FSM-280; FSM-281; FSM-284; FSM-286; FSM-288; FSM-290; FSM-292; FSM-297; FSM-298; FSM-300; FSM-303; FSM-305; FSM-308; FSM-309; FSM-311; FSM-313; FSM-314; FSM-316; FSM-318; FSM-322; FSM-323; FSM-1360; FSM-1387-2NL; FSM-MC7; FSM-MC8; FSM-MC9; FSM-MM10 and FSM-MM11, in liquid medium,
were selected.

The phage isolates may be FSM-Phage 67/33/1, FSM-Phage F, FSM-Phage 9/261, FSM-Phage F/316and FSM-Phage 73/311/2, FSM-Phage 73/261, FSM-Phage 33/33/1, FSM-Phage 28/145/2 and FSM-Phage 10/261/1.

In a preferred embodiment, the phage isolates are phages FSM-Phage 10/261/1, FSM-Phage 73/261, FSM-Phage 33/33/1, FSM-Phage 67/33/1, FSM-Phage F/316, FSM-Phage 9/261, and FSM-Phage 73/311/2 together with their corresponding propagation strains and have been deposited by way of example at the Institut Pasteur, 28 rue du Docteur Roux, F-75024 Paris CEDEX 15, FRANCE, on 14/11/2003, under the deposit number CNCM 1-3127, CNCM 1-3128, CNCM 1-3129, CNCM 1-3130, CNCM I- 3131, CNCM I-3132 and CNCM I-3133, respectively.

Advantageously, the phages deposited are able to lyse at least 80 % out of the *E*. *sakazakii* from the Nestlé Research Centre collection composed of 39 strains. This collection is representative for the *E*. *sakazakii* that can be found in food products, in particular infant formula. With the phage material according to the invention, coverage of about 97 per cent can be obtained by using a phage cocktail of at least two phages. Preferably, the phages are not able to recognize non-related bacteria such as the gram-negative lactic acid bacteria.

Thus, according to a further aspect, the invention relates to the use of at least one phage isolate of the invention, in any food product or nutritional supplement, for preventing contamination and outgrowth of *E*. *sakazakii.* Examples for food or pharmaceuticals products are milk, yoghurt, curd, cheese, fermented milks, milk based fermented products, ice-creams, fermented cereal based products, milk based powders, infant formulae or tablets, liquid suspensions, dried oral supplement, wet oral supplement, dry-tube-feeding. Methods for preparing them are common knowledge.

The composition according to the invention may also comprise usual excipients, in particular sweeteners, flavouring agents or preservatives. It can further comprise a prebiotic and/or a probiotic microorganism. The compositions of the invention may be formulated according to any one of a number of techniques that are well known to this art.

In another embodiment, a food composition containing at least one phage isolate according to the present invention is prepared. This composition may be a nutritional complete formula, an infant formula, a dairy product, a chilled or shelf stable beverage, water, a soup, a dietary supplement, a meal replacement, a nutritional bar or a confectionery. In a most preferred embodiment, phages may be added to a dry powdered infant formula.

In another embodiment, a usual food product may be enriched with at least one phage isolate according to the present invention. For example, a fermented milk, a yoghurt, a fresh cheese, a renneted milk, article of confectionery, for example a sweet or sweetened beverage, a confectionery bar, breakfast cereal flakes or bars, drinks, milk powders, soy-based products, non-milk fermented products or nutritional supplements for clinical nutrition.

Once a phage isolate according to the present invention has been selected, said phage isolate may be included alone or in a phage cocktail, into a product as mentioned above in an amount of at least about 10⁴ pfu (plaque forming unit)/ml or gram (depending if the phages are added in a dry or liquid state), and more preferably from about 10⁶ to about 10¹¹ pfu/ml or gram. The phage isolate may be prepared by spray-drying, freeze-drying, or in liquid sample container using the product as support material, e.g. milk powder for infant formula. It can also be used as a supplement to be mixed with the product before serving, as additional packaging (e.g. sachet).

Also, application of phages according to the invention or spraying or otherwise applying phages in a factory environment is performed with the objective to inhibit growth of the target bacteria. Such compositions may be prepared by conventional methods. For this application, the amount of phages is preferably of at least about 10⁶ pfu (plaque forming unit)/ml or gram, and more preferably from about 10⁶ to about 10¹¹ pfu/ml or gram.

The following examples are given by way of illustration only and in no way should be construed as limiting the subject matter of the present application. The examples are preceded by a brief description of the figures.
**Figures 1** is a diagram showing the growth of the *E*. *sakazakii* strain mixture (FSMCC # 145, 286, 290, 305, 1987/2NL) in BHI broth inoculated with 10⁸ pfu/ml phage cocktail (FSMCC-phage # 67/33/1, F/316, 9/261, 73/311/2) (●) or without phage cocktail (▲).
**Figure 2** is a diagram showing the growth of the *E*. *sakazakii* strain mixture (FSMCC # 145, 286, 290, 305, 1987/2NL) in Reconstituted Infant Formulas inoculated with 10⁸ pfu/ml phage cocktail (FSMCC-phage # 67/33/1, F/316, 9/261, 73/311/2) (●) or without phage cocktail (▲).
**Figure 3** is a diagram showing the growth of the *E*. *sakazakii* strain mixture (FSMCC # 145, 286, 290, 305, 1987/2NL) on the surface of stainless steel discs treated with 10⁸ pfu/ml phage in the phage cocktail (FSMCC-phage # 67/33/1, F/316, 9/261, 73/311/2) (●), or without phage cocktail (▲).

### Examples

### Example 1 : Selection of phages according to the invention

A collection of 114 phage isolates originating from the Nestlé Food Safety Microbiology bacteriophage collection, were tested against 39 strains of *E*. *sakazakii* originating from Food Safety Microbiology culture collection (FSMCC). The strains are listed in Table 1 and 2, respectively.

**Table 1: Phage isolates investigated**

| | Origin | FSMCC | | | | Origin | FSMCC |
|---|---|---|---|---|---|---|---|
| | | n° | | | | | n° |
| 1 | factory environment | 9/145/1 | | | 58 | Sewage (primary mud) | 64/3312 |
| 2 | " | 9/145/2 | | | 59 | '' | 64/311 |
| 3 | '' | 9/145/3 | | | 60 | Sewage (channel rejection in the lake) | 66/311/1 |
| 4 | '' | 9/145/4 | | | 61 | '' | 66/311/2 |
| 5 | '' | 9/145/5 | | | 62 | '' | 67/33/1 |
| 6 | '' | 9/261 | | | 63 | '' | 67/33/2 |
| 7 | '' | 9/261/2 | | | 64 | '' | 67/300/1 |
| 8 | '' | 10/145/1 | | | 65 | '' | 67/300/2 |
| 9 | '' | 10/145/2 | | | 66 | '' | 67/311/1 |
| 10 | '' | 10/145/3 | | | 67 | '' | 67/311/2 |
| 11 | '' | 10/145/4 | | | 68 | Sewage (arrival of water | 61/MC9/1 |
| | | | | | | before filtration on sand) | |
| 12 | '' | 10/261/1 | | | 69 | '' | 61/MC9/2 |
| 13 | '' | 10/261/2 | | | 70 | '' | 61/MC9/3 |
| 14 | '' | 10/261/3 | | | 71 | Sewage (primary mud) | 64/MC9/1 |
| 15 | '' | 10/261/4 | | | 72 | '' | 64/MC9/2 |
| 16 | '' | 10/261/5 | | | 73 | Sewage (channel rejection in the lake) | 66/MC9/1 |
| 17 | Waste water | 16/145 | | | 74 | '' | 66/MC9/2 |
| 18 | Factory environment | 22/145/1 | | | 75 | '' | 67/MC9/1 |
| 19 | '' | 22/14512 | | | 76 | '' | 67/MC9/2 |
| 20 | '' | 22/145/3 | | | 77 | Sewage (entry of water) | 73/33/1 |
| 21 | '' | 22/145/4 | | | 78 | '' | 73/33/2 |
| 22 | '' | 22/145/4 | | | 79 | '' | 73/261/1 |
| 23 | '' | 23/16 | | | 80 | '' | 73/26112 |
| 24 | '' | 23/145/1 | | | 81 | '' | 73/300/1 |
| 25 | '' | 23/145/2 | | | 82 | '' | 73/300/2 |
| 26 | '' | 23/145/3 | | | 83 | '' | 73/311/1 |
| 27 | '' | 23/145/4 | | | 84 | '' | 73/311/2 |
| 28 | '' | 23/145/5 | | | 85 | '' | 73/MC9/1 |
| 29 | '' | 23/1387 | | | 86 | '' | 73/MC9/2 |
| 30 | '' | 28/16 | | | 87 | Sewage (exit of water) | 74/MC9/1 |
| 31 | '' | 28/145/1 | | | 88 | '' | 74/MC9/2 |
| 32 | '' | 28/145/2 | | | 89 | Sewage (biological water) | 75/311/1 |
| 33 | '' | 33/33/1 | | | 90 | '' | 75/311/2 |
| 34 | '' | 33/33/2 | | | 91 | '' | 75/MC9/1 |
| 35 | '' | 33/33/3 | | | 92 | '' | 75/MC9/2 |
| 36 | '' | 33/33/4 | | | 93 | Sewage (entry of water) | 76/33/1 |
| 37 | '' | 33/33/5 | | | 94 | '' | 76/3 3/2 |
| 38 | '' | 46/MC9/1 | | | 95 | '' | 76/311/1 |
| 39 | '' | 46/MC9/2 | | | 96 | '' | 76/311/2 |
| 40 | '' | 58/MC9/1 | | | 97 | '' | 76/311/3 |
| 41 | '' | 58/MC9/2 | | | 98 | '' | 76/311/4 |
| 42 | '' | 58/MC9/3 | | | 99 | '' | 76/311/5 |
| 43 | Sewage | 61/33/5 | | | 100 | '' | 76/MC9/1 |
| 44 | '' | 61/33/6 | | | 101 | '' | 76/MC9/2 |
| 45 | '' | 61/33/7 | | | 102 | Sewage (exit of water) | 77/33/1 |
| 46 | '' | 61/33/8 | | | 103 | '' | 77/33/2 |
| 47 | '' | 61/300 | | | 104 | '' | 77/311/1 |
| 48 | '' | 61/300/1 | | | 105 | '' | 77/311/2 |
| 49 | '' | 61/300/2 | | | 106 | '' | 77/MC9/1 |
| 50 | '' | 61/311/1 | | | 107 | '' | 77/MC9/2 |
| 51 | '' | 61/311/2 | | | 108 | Sewage (biological water) | 78/33/1 |
| 52 | '' | 61/311/3 | | | 109 | '' | 78/33/2 |
| 53 | '' | 61/311/4 | | | 110 | '' | 78/311/1 |
| 54 | '' | 61/311/5 | | | 111 | '' | 78/311/2 |
| 55 | '' | 62/300/1 | | | 112 | '' | 78/MC9/1 |
| 56 | '' | 62/300/2 | | | 113 | '' | 78/MC9/2 |
| 57 | Sewage (primary mud) | 64/33/1 | | | 114 | Factory environment | F/316 |

**Table 2: Enterobacter sakazakii strains**

| **FSM-Code** | **Origin** | | | **FSM- Code** | **Origin** |
|---|---|---|---|---|---|
| Mat-16 (16) | Finished product: cereals | | | 300 | Milk Factory environment |
| 33 | Infant formula | | | 303 | Milk Factory environment |
| 261 | Munich University | | | 305 | Milk Factory environment |
| 265 | Unknown | | | 308 | Milk Factory environment |
| 266 | Unknown | | | 309 | Milk Factory environment |
| 269 | Milk Factory environment | | | 311 | Milk Factory environment |
| 270 | Milk Factory environment | | | 313 | Milk Factory environment |
| 271 | Milk Factory environment | | | 314 | Milk Factory environment |
| 272 | Milk Factory environment | | | 316 | Milk Factory environment |
| 273 | Milk Factory environment | | | 318 | Milk Factory environment |
| 274 | Milk Factory environment | | | 322 | Milk Factory environment |
| 280 | Milk Factory environment | | | 323 | Milk Factory environment |
| 281 | Milk Factory environment | | | 1360 | Milk Factory environment |
| 284 | Milk Factory environment | | | 1387-2NL | Milk Factory environment |
| 286 | Milk Factory environment | | | MC7 | Wageningen University |
| 288 | Milk Factory environment | | | MC8 | Wageningen University |
| 290 | Milk Factory environment | | | MC9 | Wageningen University |
| 292 | Milk Factory environment | | | MM10 | Wageningen University |
| 297 | Milk Factory environment | | | MM11 | Wageningen University |
| 298 | Milk Factory environment | | | | |

### SCREENING ON SOLID MEDIUM

### Isolation of plagues and phage amplification

Sampling:
Take sewage water samples (approx. 50 ml) for the research for bacteriophages. Measure the pH of each sample using pH paper strips (if pH lower than 5, to adjust to 7 with NaOH 1N).

The samples are then submitted to the following steps:
1. Centrifugation 10 min at 2500g (to eliminate the large remains).
2. Filtration of the supernatant using a filter of 0.20 µm (to eliminate bacteria)
3. Perform the tests as follows or keep samples at 4°C for 1 or 2 days MAXIMUM.

For the isolation of bacteriophages, the above pre-treated samples have been tested in parallel with or without pre-amplification. Once the presence of phage is detected, they are amplified and stored.

### Screening without pre-amplification:

### Step 1: Preparation of the bacterial culture:

A culture of *E. sakazakii* having grown overnight at 30°C in Brain Heart Infusion (BHI, Oxoid CM225) is prepared, and then 0.5 ml of this culture is transferred in 4ml of fresh BHI and incubated 1h at 40°C.

### Step 2: Add samples to bacteria:

Add in a tube containing 3ml soft BHI agar (BHI broth + 0.6% agar) maintained at 45 ± 1 °C, 100 µl of the *E*. *sakazakii* culture (prepared as above) + 100 µl of treated sample to be tested.

### Step 3: Inoculation of Petri dishes:

Mix the tube content with a Vortex (± 2sec.). Then, pour the whole tube content on the surface of a Petri dish containing a thin layer of BHI agar and let solidify on the bench. Incubate inverted plates overnight at 30°C.

### Step 4: Interpretation of results:

Note the presence or absence of plaques of lysis on each Petri dish.

### Step 5: Controls

Prepare a positive control (*E. sakazakii* strain n° 261 + active phage against this strain at a concentration of 2-3 log pfu/ml): Apply steps 1 to 4.

Prepare a negative control without phage added: Apply steps 1 to 4 without addition of phage or sample to be tested.

### Screening with pre-amplification:

### Step 1- Day 1: Preparation of the bacterial culture:

A culture of *E. sakazakii* having grown overnight at 30°C in Brain Heart Infusion (BHI, Oxoid CM225) is prepared, then 0.5 ml of this culture is transferred in 4ml of fresh BHI and incubated 1h at 40°C.

### Step 2 - Day 1: Amplification of phage in the sample:

In a tube containing 4ml BHI broth, 1 100µl of the *E. sakazakii* culture (prepared as in step 1) + 1 ml of treated sample to be tested, are added and incubated 3-5h at 30°C under agitation at 150-200 rpm. They are left overnight on the bench.

### Step 3 - Day 2: Centrifugation 10 min at 2500 rpm

### Step 4 - Day 2: Preparation of the bacterial culture:

A culture of *E. sakazakii* having grown overnight at 30°C in Brain Heart Infusion (BHI, Oxoid CM225) is prepared, then 0.5 ml of this culture is transferred in 4ml of fresh BHI and incubated 1h at 40°C.

### Step 5 - Day 2: Add samples to bacteria:

Add in a tube containing 3ml soft BHI agar (BHI broth + 0.6% agar) maintained at 45 ± 1°C, 100 µl of the *E*. *sakazakii* culture (prepared as above) + 100 µl of sample obtained in step 3.

### Step 6 - Day 2: Inoculation of Petri dishes:

Mix the tube content with a Vortex (± 2sec.). Then, pour the whole tube content on the surface of a Petri dish containing a thin layer of BHI agar and let solidify on the bench. Incubate inverted plates overnight at 30°C.

### Step 7 - Day 3: Interpretation of results: Note the presence or absence of plaques of lyses on each Petri dish.

When the presence of phage is detected, they are amplified and stored.

### Amplification of a lyses plaque

### Step 1: Preparation of the bacterial culture:

A culture of *E. sakazakii* having grown overnight at 30°C in Brain Heart Infusion (BHI, Oxoid CM225) is prepared, then 0.5 ml of this culture is transferred in 4ml of fresh BHI and incubated 1h at 40°C.

### Step 2: Amplification of the pfu:

In a tube containing 4ml BHI broth, 100 gel of the *E. sakazakii* culture (prepared as in step 1) + 1 isolated pfu (cut from Agar) are added and incubated 3-5h at 30°C under agitation at 150-200 rpm. Tubes are left overnight on the bench.

### Step 3: Centrifugation 10 min at 2500rpm.

### Step 4: Filtration of the supernatant using a filter of 0.20 µm (to eliminate bacteria)

### Enumeration of phages

### Step 5: Preparation of the bacterial culture:

A culture of *E*. *sakazakii* having grown overnight at 30°C in Brain Heart Infusion (BHI, Oxoid CM225) is prepared and then 0.5 ml of this culture is transferred in 4ml of fresh BHI and incubated 1h at 40°C.

### Step 6: Add phages to bacteria:

Add in a tube containing 3ml soft BHI agar (BHI broth + 0.6% agar) maintained at 45 ± 1°C, 100 µl of the *E*. *sakazakii* culture (prepared as above) + 100 µl of serially diluted sample obtained in step 4.

### Step 7: Inoculation of Petri dishes:

Mix the tube content with a Vortex (± 2sec.) and pour the whole tube content on the surface of a Petri dish containing a thin layer of BHI agar. Then, let solidify on the bench and incubate inverted plates overnight at 30°C.

### Step 8: Interpretation of results:

Enumerate lyses plaques on each Petri dish.

### Storage after amplification

### Step 1: Preparation of aliquots:

Distribute into cryo-tubes approx. 1.5ml of the filtered amplified phage solution (obtained in step 4 of the "Amplification of one lyses plaque" paragraph)

### Step 2: Addition of glycerol:

In each tube 15% of sterile glycerol is added and mixed quickly using a Vortex.

### Step 3: Quickly freeze the tubes in liquid nitrogen

### Step 4: Storage of the tubes in a freezer at -20°C

### SCREENING IN LIQUID MEDIUM

### Step 1, Day 0: Preparation of the bacterial culture:

A culture of *E. sakazakii* having grown overnight at 30°C in Brain Heart Infusion (BHI, Oxoid CM225) is prepared.

### Step 2, Day 1: Preparation of the bacterial culture:

Transfer 0.1 ml of this culture in 4ml of fresh BHI and incubated 6h at 40°C (stationary growth phase of cells). Dilute cells up to 3log cfu/ml in Tryptone Salt (TS, Oxoid L42).

### Step 3, Day 1: Microtiterplate assay

Effectiveness of phages against *E*. *sakazakii* was investigated in a microtiterplate assay. Each plate was set up with each of the test microorganisms as follows: external wells, 200 µl of sterile BHI (blank control), other wells, 20 µl of diluted *E*. *sakazakii* prepared in step 2. Store overnight microtiterplates at 4°C.

### Step 4, Day 2: Microtiterplate assay

Leave the microtiterplates from the refrigerator. Add in negative control wells, 180µl BHI and in test wells, 160µl BHI plus 20µl phage suspension (final concentration of 8 log pfu/ml). Read at 620nm the optical density (time zero) of the microtiterplate by using a microplate reader Multiskan MCC340. Plates are then incubated at 37°C in ambient air and OD₆₂₀ₙₘ was read after 6h30 and 8h30 incubation.

### Step 5, Day 3: Microtiterplate assay

After the last reading, plates are left at ambient temperature for one total duration of 24h. After which the OD₆₂₀ₙₘ is last once read.

### RESULTS

Following to the test on solid medium, phage isolates that are active against at least 9 out of the 39 *E*. *sakazakii* strains selected from the group :FSM-16; 33; 261; 265; 266,269;270;271;272;273;274;280;281;284;286;288;290;292;297;298;300; 303; 305; 308; 309; 311; 313; 314; 316; 318; 322; 323; 1360; 1387/2NL; MC7; MC8; MC9; MM10 and MM11, are selected. Thus, the 11 phage isolates (including the phages FSM-Phage 67/33/1, FSM-Phage F/316, FSM-Phage 9/261, and FSM-Phage 73/311/2) are presented in Table 3. Some phage isolates proved to be efficient against at least 20 out of the 39 bacterial strains.

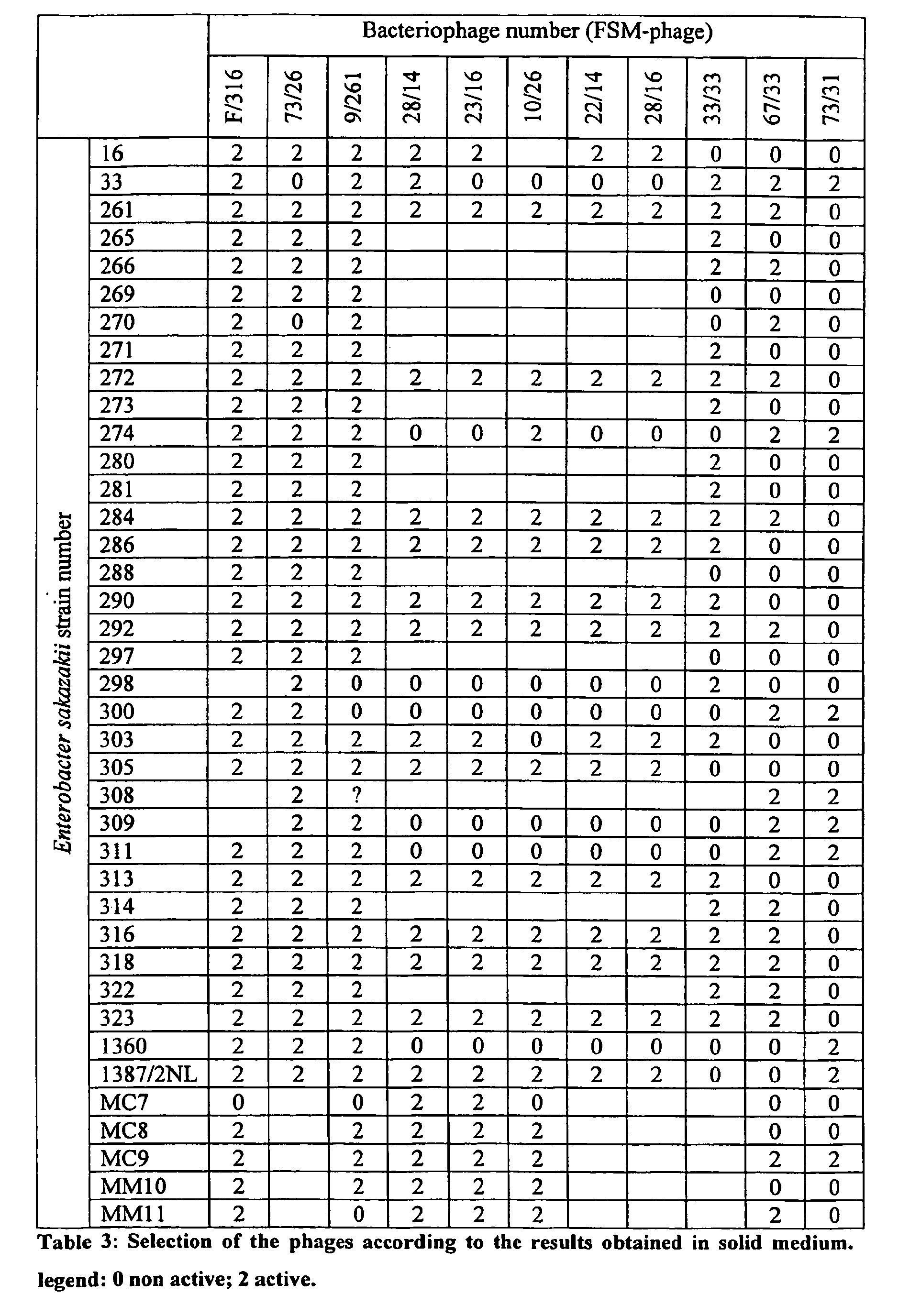

The said phages were further tested on liquid medium in order to select the phage isolates according to the present invention. The results are then presented in Table 4.

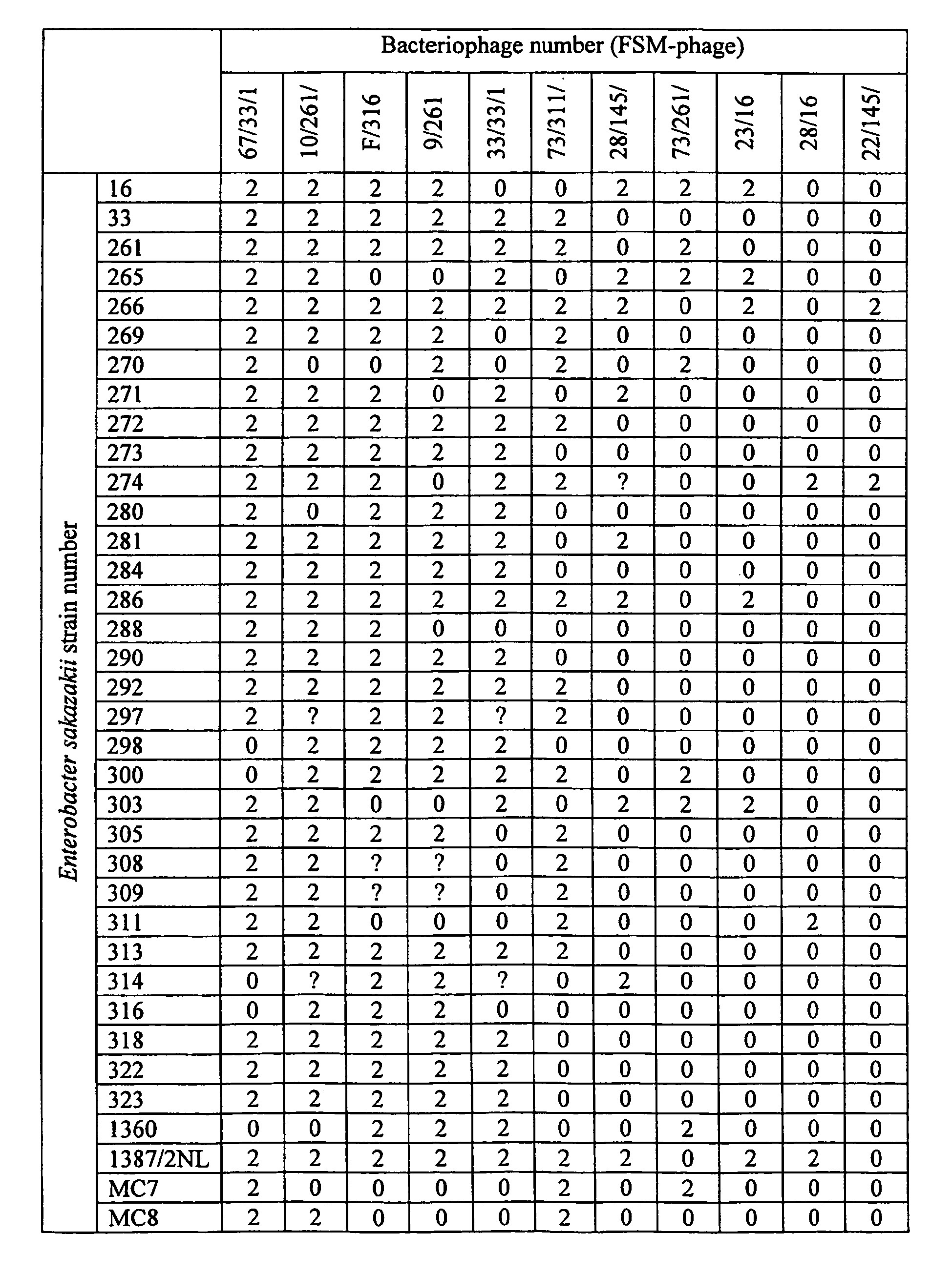

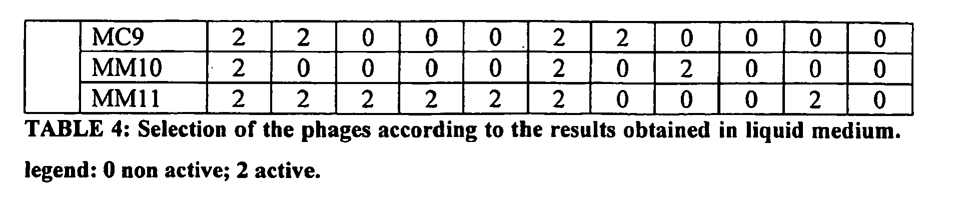

As may be derived from Table 4, some phage isolate recognize more than 80 % of the 39 *E. sakazakii* strains. Other isolates, however, recognize not even 10 % of the strains tested. The phages isolates that are further active in liquid medium against at least 9 out of the 39 *E. sakazakii* strains selected from the group : FSM-16; FSM-33; FSM-261; FSM-265; FSM-266, FSM-269; FSM-270; FSM-271; FSM-272; FSM-273; FSM-274; FSM-280; FSM-281; FSM-284; FSM-286; FSM-288; FSM-290; FSM-292; FSM-297; FSM-298; FSM-300; FSM-303; FSM-305; FSM-308; FSM-309; FSM-311; FSM-313; FSM-314; FSM-316; FSM-318; FSM-322; FSM-323; FSM-1360; FSM-1387-2NL; FSM-MC7; FSM-MC8; FSM-MC9; FSM-MM10 and FSM-MM11, are selected phage isolates that can be used in the present invention. Preferably, the following 8 phage isolates FSM-Phage 67/33/1 (CNCM I-3130), FSM-Phage F/316 (CNCM I-3131), FSM-Phage 9/261 (CNCM I-3132), and FSM-Phage 73/311/2 (CNCM I-3133), FSM-Phage 73/261(CNCM I-3128), FMS 33/33/1, (CNCM I-3129) FSM-Phage 28/145/2, FSM-Phage 10/261/1 (CNCM I-3127).

It will be appreciated that a skilled person may well examine and select other phage isolates according to the present invention, by subjecting them to the screening tests on solid and liquid medium as detailed above.

### Example 2: Evaluation of bacteriophage cocktail efficacy against a mix of various strains of Enterobacter sakazakii in BHI and Reconstituted Infant Formula (RIF)

In order to evaluate the efficacy of the phage cocktail against *E. sakazakii* strain mixture, the following experiment was carried out.

A mixture of the *E. sakazakii* strains FSM-145, 286, 290, 305 and 1387/2NL as described in example 1 and in P. Breeuwer et al., 2003, Journal of Applied Microbiology 95: 967-973, is used. Individual overnight culture (18h at 30°C) of these strains is diluted 1:1 in fresh BHI and incubated 2h at 30°C. A mix of these strains is carried-out by mixing equal quantities of each stock. Then, the BHI broth or RIF are inoculated with 100 µl of the *E. sakazakii* mix (prepared as described above) so that the final concentration is of about 10 cfu/ml.

The phage isolate cocktail containing FSM_phage 67/33/1 (CNCM I-3130), FSM_phage F/316 (CNCM I-3131), FSM_phage 9/261 (CNCM I-3132) and FSM_phage 73/311/2 (CNCM I-3133) has been tested. For this, a variable amount of each phage is added to the tubes containing *E. sakazakii* mix to reach a final concentration of about 8 log pfu/ml. Negative control without addition of phage (BHI or RIF 9.9ml + *E. sakazakii* mix 100µl) is also included.

All inoculates are incubated at 30°C and samples are taken at various time intervals: 0 - 2 - 4 - 6h. For each sample, the number of bacteria present in the medium was determined by standard plate counting method.

### Results:

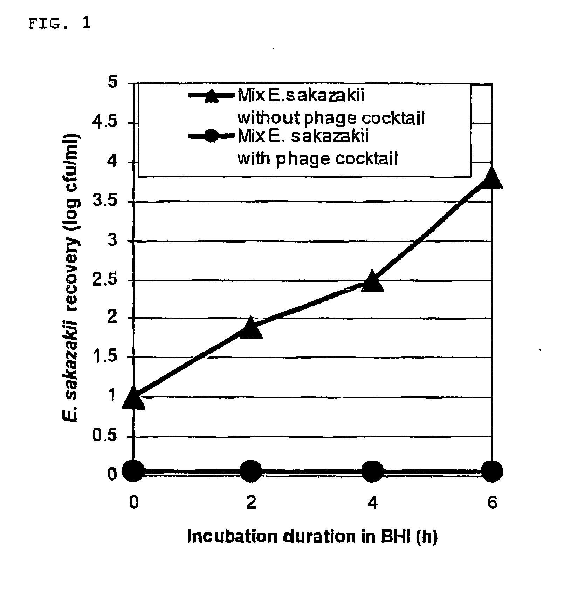

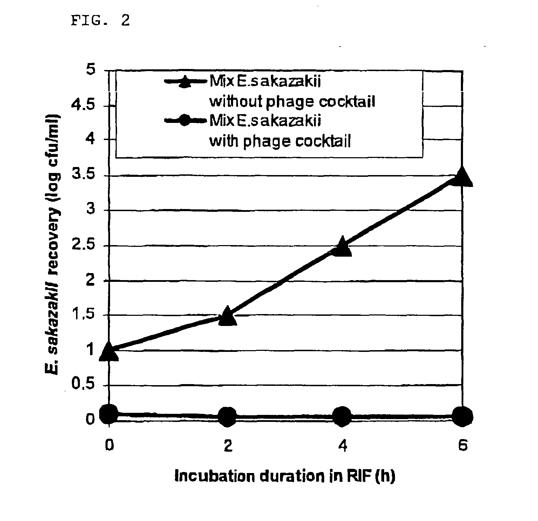

The results are presented in figures 1 and 2. It shows that a bacterial mixture will grow out to high number in the absence of phage cocktail whereas no growth is observed in the presence of the phage cocktail for a period of minimum 6 hours and up to 24 hours.

In conclusion, these experiments demonstrate that adding bacteriophage preparation according to the present invention against *E*. *sakazakii* to reconstituted infant formulas (RIF) should prevent outgrowth of *E. sakazakii* for at least 6 hours.

### Example 3: Effect on bacteriophage preparation for environment sanitation.

In order to evaluate the efficacy of the phage cocktail on artificially contaminated surfaces by a *E. sakazakii* strain mixture, the following experiment was carried out.

### Step 1: Preparation of bacteria cells

A mixture of the *E. sakazakii* strains (FSM-145, 286, 290, 305 and 1387/2NL (see examples 1 and 2)) is used. Individual overnight culture (18h at 30°C) of these strains is centrifuged at 3000rpm for 10min at 4°C. Cell pellet is harvested and diluted in BHI to reach a cell concentration of about 8 log cfu/ml. A mix of these strains is carried-out by mixing equal quantities of each stock.

### Step 2: Adhesion

Stainless steel disc of 1cm² are plunged in the bacterial solution prepared as described in step 1. Leave at room temperature under gentle shaking for 1h.

### Step 3: Washing and Drying

To remove unattached cells, stainless steel discs are withdrawn and washed 3 times in Phosphate Buffer solution (PBS: pH 7.0, Merck). The excess of liquid is absorbed with Watman paper, the discs are then dried during 30min under laminar flow. To determine the level of attached cells, step 6 is applied on some discs.

### Step 4: Addition of phage cocktail

Half of stainless steel discs are plunged in a 8 log pfu/ml phage cocktail solution containing the following phages: FSM_phage 67/33/1 (CNCM I-3130), FSM_phage F/316 (CNCM I-3131), FSM_phage 9/261 (CNCM I-3132) and FSM_phage 73/311/2 (CNCM I-3133). Leave the container at room temperature under gentle shaking for 6 hours. Withdraw some discs at various intervals for bacteria enumeration. In parallel, controls without phage added are carried-out.

### Step 5: Washing

At defined intervals some discs are withdrawn, washed 3 times in sterile PBS and dry quickly on sterile absorbent paper.

### Step 6: Cell detachment and enumeration

After step 5, put each disc into tubes containing 9ml TS. Tubes are placed into sonication bath (filled with water and ice mix) for 1min at 50KHz afterwards; tubes are agitated for 10 seconds by using a Vortex. Cell enumeration is performed using a standard plating method.

### Results:

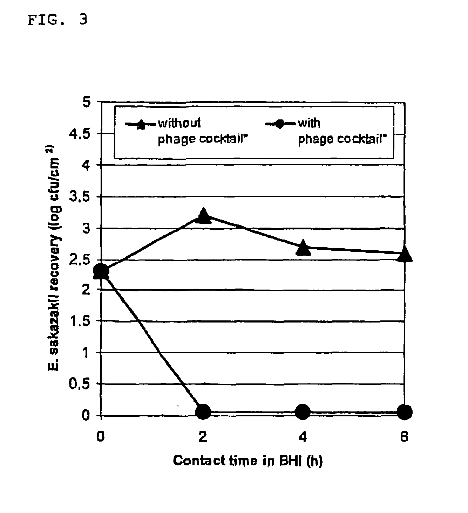

The results are presented in Figure 3 which demonstrates that the *E. sakazakii* bacteria in the strain mixture (FSMCC # 145, 286, 290, 305, 1987/2NL) can not be recovered anymore from the surface of stainless steel discs treated with 10⁸ pfu/ml phage in the phage cocktail (FSMCC-phage # 67/33/1, F/316, 9/261, 73/311/2), whereas without phage cocktail the bacteria remain viable and can even grow slightly.

In conclusion, the treatment of stainless steel surfaces with at least 10⁸ phages per ml result in killing of the *E. sakazakii* bacteria.

## Claims

1. A phage isolate with substantial lytic potential towards *E*. *sakazakii* selected from the group consisting of CNCM I-3127, CNCM I-3128, CNCM I-3129, CNCM I-3130, CNCM I-3131, CNCM I-3132, and CNCM I-3133.

2. A phage cocktail containing at least one phage isolate according to claim 1.

3. A food product containing at least one phage isolate or cocktail with substantial lytic potential towards *E. sakazakii,* wherein the phage isolate or cocktail is according to claims 1 to 2, said food being in the form of a milk based product, a nutritional composition, a pet food, a dietary supplement or any food at risk of contamination by *E. sakazakii.*

4. A food product according to claim 3, wherein the milk based product is a fermented milk, a yoghurt, a curd, a cheese, a fresh cheese, a fermented product, a renneted milk, a drink, a milk based powder, an infant formula or a dried powdered infant formula.

5. A food product according to one of claims 3 to 4, in which the phage isolate or cocktail is in an amount effective to prevent contamination and outgrowth of *E*. *sakazakii* in said product.

6. A food product according to claim 5, wherein the phage isolate is present in an amount of at least 1.10⁴ pfu per ml, or gram if solid, of the food product.

7. An agent for decontamination of food product by prevention of contamination and outgrowth of *E*. *sakazakii* and sanitation of factory environment, which comprises an effective amount of at least one phage isolate or cocktail according to one of claims 1 to 2.

8. An agent according to claim 7, wherein the phage isolate is present in an amount of at least 1.10⁴ pfu per ml, or gram if solid.

9. An agent according to one of claims 7 or 8, wherein the food product is a milk based product, such as a fermented milk, a yoghurt, curd, cheese, fresh cheese, milk based fermented products, renneted milk, drinks, milk based powders, an infant formula, a dried powdered infant formula; a non-milk fermented product; a soy-based product, a nutritional formula, a dairy product, a chilled or shelf stable beverage, water, soup, a dietary supplement, a meal replacement, a nutritional bar or a confectionery, a pet food product, a sweet or sweetened beverage, an ice-cream, a confectionery bar, breakfast cereal flakes or bars or nutritional supplement for clinical nutrition or any food at risk of contamination by *E. sakazakii.*

10. Use of at least one phage isolate or cocktail according to one of claims 1 to 2, for decontamination of food products and sanitation of factory environment, wherein the food product is a milk based product, such as a fermented milk, a yoghurt, a curd, a cheese, a fresh cheese, milk based fermented products, renneted milk, drinks, milk based powders, an infant formula, a dried powdered infant formula; a non-milk fermented product; a soy-based product, a nutritional formula, a dairy product, a chilled or shelf stable beverage, water, soup, a dietary supplement, a meal replacement, a nutritional bar or a confectionery, a pet food product, a sweet or sweetened beverage, an ice-cream, a confectionery bar, breakfast cereal flakes or bars or nutritional supplement for clinical nutrition or any food at risk of contamination by *E. sakazakii.*

11. Use of at least one phage isolate according to one of claims 1 to 2, in the preparation of a composition intended to prevent or treat infections caused by *E. sakazakii* in humans or animals, in which the phage isolate is present in an amount efficient to prevent contamination and outgrowth of *Enterobacter sakazakii.*

12. The use according to one of claims 10 to 11, wherein the phage isolate or cocktail is used in an amount of at least 1.10⁴ pfu per ml.

## Patentansprüche

1. Phagenisolat mit im Wesentlichen lytischem Potenzial gegenüber *E*. *Sakazakii* ausgewählt aus der Gruppe bestehend aus CNCM I-3127, CNCM I-3128, CNCM I-3129, CNCM I-3130, CNCM I-3131, CNCM I-3132, und CNCM I-3133.

2. Phagen-Cocktail enthaltend mindestens ein Phagenisolat gemäß Anspruch 1.

3. Lebensmittelprodukt enthaltend mindestens ein Phagenisolat oder -cocktail mit im Wesentlichen lytischem Potenzial gegenüber *E*. *Sakazakii,* worin das Phagenisolat oder -cocktail gemäß Ansprüchen 1 bis 2 ist, wobei das Lebensmittel in der Form eines auf Milch basierenden Produktes, einer Nahrungsmittelzusammensetzung, eines Haustiernahrungsmittels, einer Diätzusammensetzung oder eines beliebigen Lebensmittels mit Risiko der Kontamination durch *E*. *Sakazakii* ist.

4. Lebensmittelprodukt gemäß Anspruch 3, worin das auf Milch basierende Produkt fermentierte Milch, Joghurt, Quark, Käse, Frischkäse, ein fermentiertes Produkt, Lab behandelte Milch, ein Drink, ein auf Milch basierendes Pulver, Säuglingsnahrung oder getrocknete pulverisierte Säuglingsnahrung ist.

5. Lebensmittelprodukt gemäß einem der Ansprüche 3 oder 4, worin das Phagenisolat oder -cocktail in einer Menge ist, die zum Verhindern der Kontamination und des Auswuchs von *E*. *Sakazakii* in dem Produkt effektiv ist.

6. Lebensmittelprodukt gemäß Anspruch 5, worin das Phagenisolat in einer Menge von mindestens 1.10⁴ pfu pro ml, oder Gramm, wenn fest, des Lebensmittelproduktes vorhanden ist.

7. Mittel zur Dekontamination eines Lebensmittelproduktes durch Verhinderung der Kontamination und Auswuchs von *E*. *Sakazakii* und Reinigung der Fabrik Umgebung, welches eine effektive Menge von mindestens einem Phagenisolat oder -cocktail gemäß einem der der Ansprüche 1 bis 2 umfasst.

8. Mittel gemäß Anspruch 7, worin das Phagenisolat in einer Menge von mindestens 1.10⁴ pfu pro ml oder Gramm, wenn fest, vorhanden ist.

9. Mittel gemäß einem der Ansprüche 7 oder 8, worin das Lebensmittelprodukt ein auf Milch basierendes Produkt, wie etwa fermentierte Milch, Joghurt, Quark, Käse, Frischkäse, auf Milch basierende fermentierte Produkte, Lab behandelte Milch, Drinks, Milch basierte Pulver, Säuglingsnahrung, getrocknete pulverisierte Säuglingsnahrung; ein nicht-Milch fermentiertes Produkt; ein Soja-basiertes Produkt, eine Nahrungsmittelformel, ein Molkereiprodukt, ein gekühltes oder haltbares Getränk, Wasser, Suppe, ein Diätergänzungsmittel, ein Mahlzeitersatz, ein Nahrungsmittelriegel oder eine Süßware, ein Haustiernahrungsprodukt, eine süßes oder gesüßtes Getränk, eine Eiscreme, ein Süßwarenriegel, Frühstückscerealienflocken oder -riegel oder Nahrungsergänzung für die klinische Ernährung oder ein beliebiges Lebensmittel mit Risiko der Kontamination durch *E*. *Sakazakii* ist.

10. Verwendung mindestens eines Phagenisolats oder -cocktails gemäß einem der Ansprüche 1 bis 2, zur Dekontamination von Lebensmittelprodukten und Reinigung der Fabrikumgebung, worin das Lebensmittelprodukt ein auf Milch basierendes Produkt, wie etwa fermentierte Milch, Joghurt, Quark, Käse, Frischkäse, auf Milch basierende fermentierte Produkte, Lab behandelte Milch, Drinks, Milch basierte Pulver, Säuglingsnahrung, getrocknete pulverisierte Säuglingsnahrung; ein nicht-Milch fermentiertes Produkt; ein Soja-basiertes Produkt, eine Nahrungsmittelformel, ein Molkereiprodukt, ein gekühltes oder haltbares Getränk, Wasser, Suppe, ein Diätergänzungsmittel, ein Mahlzeitersatz, ein Nahrungsmittelriegel oder eine Süßware, ein Haustiernahrungsprodukt, eine süßes oder gesüßtes Getränk, eine Eiscreme, ein Süßwarenriegel, Frühstückscerealienflocken oder -riegel oder Nahrungsergänzung für die klinische Ernährung oder ein beliebiges Lebensmittel mit Risiko der Kontamination durch *E*. *Sakazakii* ist.

11. Verwendung mindestens eines Phagenisolats gemäß einem der Ansprüche 1 bis 2, bei der Herstellung einer Zusammensetzung, die zur Verhinderung oder Behandlung von Infektionen, die durch *E*. *Sakazakii* in Menschen oder Tieren verursacht werden, beabsichtig ist, worin das Phagenisolat in einer Menge vorhanden ist, die zur Verhinderung von Kontamination und Auswuchs von Enterobacter Sakazakii effektiv ist.

12. Verwendung gemäß einem der Ansprüche 10 bis 11, worin das Phagenisolat odercocktail in einer Menge von mindestens 1.10⁴ pfu pro ml verwendet wird.

## Revendications

1. Un isolat de phage avec essentiellement un potentiel lytique envers *E*. *Sakazakii* sélectionné parmi le groupe constitué de CNCM I-3127, CNCM I-3128, CNCM 1-3129, CNCM I-3130, CNCM I-3131, CNCM I-3132, et CNCM I-3133.

2. Un cocktail de phages contenant au moins un isolat de phage selon la revendication 1.

3. Un produit alimentaire contenant au moins un isolat de phage ou un cocktail de phages avec essentiellement un potentiel lytique envers *E*. *Sakazakii,* l'isolat de phage ou le cocktail de phages étant selon la revendication 1 ou 2, ledit aliment étant de la forme d'un produit à base de lait, une composition nutritionnelle, un aliment pour animal domestique, un supplément diététique ou un n'importe quel aliment ayant un risque de contamination par *E*. *Sakazakii.*

4. Un produit alimentaire selon la revendication 3,
dans lequel le produit à base de lait est du lait fermenté, du yogourt, du lait caillé, du fromage, du fromage frais, un produit fermenté, du lait emprésuré, une boisson, une poudre à base de lait, une formule pour nourrisson ou une formule pour nourrisson en poudre et séchée.

5. Un produit alimentaire selon l'une des revendications 3 à 4,
dans lequel l'isolat de phage ou le cocktail de phages est dans une quantité effective pour prévenir la contamination et l'excroissance de *E*. *Sakazakii* dans ledit produit.

6. Un produit alimentaire selon la revendication 5,
dans lequel l'isolat de phage est présent dans une quantité d'au moins 1.10⁴ pfu par ml, ou gramme si solide, du produit alimentaire.

7. Un agent pour la décontamination de produit alimentaire par la prévention de la contamination et de l'excroissance de *E*. *Sakazakii* et l'assainissement de milieu industriel, qui comprend une quantité effective d'au moins un isolat de phage ou cocktail de phages selon l'une des revendications 1 à 2.

8. Un agent selon la revendication 7,
dans lequel l'isolat de phage est présent dans une quantité d'au moins 1.10⁴ pfu par ml, ou gramme si solide.

9. Un agent selon l'une des revendications 7 ou 8,
dans lequel le produit alimentaire est un produit à base de lait, tel que du lait fermenté, du yogourt, du lait caillé, du fromage, du fromage frais, des produits fermentés à base de lait, du lait emprésuré, des boissons, des poudres à base de lait, une formule pour nourrisson, une formule pour nourrisson en poudre et séchée ; un produit fermenté non-laitier ; un produit à base de soja, une formule nutritionnelle, un produit laitier, une boisson rafraîchie ou réfrigérée ou de longue conservation, eau, soupe, un supplément diététique, un substitut de repas, une barre nutritionnelle ou une confiserie, un produit alimentaire pour animal domestique, une boisson sucrée ou édulcorée, une crème glacée, une barre de confiserie, des flocons de céréales pour petit-déjeuner ou des barres ou un supplément nutritionnel pour alimentation clinique or n'importe quel aliment à risque de contamination par *E*. *Sakazakii.*

10. Utilisation d'au moins un isolat de phage ou cocktail de phages selon l'une des revendications 1 à 2, pour la décontamination de produits alimentaires et l'assainissement de milieu industriel, le produit alimentaire étant un produit à base de lait, tel que du lait fermenté, du yogourt, du lait caillé, du fromage, du fromage frais, des produits fermentés à base de lait, du lait emprésuré, des boissons, des poudres à base de lait, une formule pour nourrisson, une formule pour nourrisson en poudre et séchée ; un produit fermenté non-laitier ; un produit à base de soja, une formule nutritionnelle, un produit laitier, une boisson rafraîchie ou réfrigérée ou de longue conservation, eau, soupe, un supplément diététique, un substitut de repas, une barre nutritionnelle ou une confiserie, un produit alimentaire pour animal domestique, une boisson sucrée ou édulcorée, une crème glacée, une barre de confiserie, des flocons de céréales pour petit-déjeuner ou des barres ou un supplément nutritionnel pour alimentation clinique or n'importe quel aliment à risque de contamination par *E*. *Sakazakii.*

11. Utilisation d'au moins un isolat de phage selon l'une des revendications 1 à 2, dans la préparation d'une composition destinée à prévenir ou traiter des infections causées par *E. Sakazakii* chez les humains ou les animaux, l'isolat de phage étant présent dans une quantité effective pour prévenir la contamination et l'excroissance de *E*. *Sakazakii.*

12. L'utilisation selon l'une des revendications 10 à 11,
dans lequel l'isolat de phage ou le cocktail de phages est utilisé dans une quantité d'au moins 1.10⁴ pfu par ml.
